Europäisches Patentamt

European Patent Office

Office européen des brevets

(19)

(11) Publication number: **0 223 254 B1**

## (12) EUROPEAN PATENT SPECIFICATION

(45) Date of publication of patent specification: **12.06.91**

(51) Int. Cl.⁵: **A61K 37/64, C12Q 1/37**

(21) Application number: **86116096.8**

(22) Date of filing: **20.11.86**

(54) Use of preparations for the treatment and prevention of epithelial destruction.

(30) Priority: **22.11.85 FI 854634**

(43) Date of publication of application:
**27.05.87 Bulletin 87/22**

(45) Publication of the grant of the patent:
**12.06.91 Bulletin 91/24**

(84) Designated Contracting States:
**BE CH DE FR GB IT LI LU NL SE**

(56) References cited:
**EP-A- 0 132 732**
**EP-A- 0 189 784**
**US-A- 4 276 284**

**INVESTIGATIVE OPHTHALMOLOGY & VISUAL SCIENCE, (USA), vol. 18, no.8, 1979; L.SKOZA et al., pp. 827-830**

**CHEMICAL ABSTRACTS , vol. 80, no. 3, 21 January 1974, Columbus, OH (US); M.B.BERMAN et al., p. 294, no. 13305e**

(73) Proprietor: **HUHTAMÄKI OY**
**Box 415**
**SF-20101 Turku(FI)**

(72) Inventor: **Salonen, Eeva-Marjatta**
**Kuutamokatu 4 B**
**SF-02210 Espoo(FI)**

(74) Representative: **Leiser, Gottfried, Dipl.-Ing. et al**
**Patentanwälte Prinz, Leiser, Bunke & Partner**
**Manzingerweg 7**
**W-8000 München 60(DE)**

## Description

This invention relates to the use of a plasmin inhibitor for the manufacture of a pharmaceutical preparation for the treatment of corneal lesions. The invention relates also to a method for determining the presence of corneal lesions.

It is known to treat epithelial lesions such as corneal lesions using local antibiotics according to sensitivity tests of conjunctival or corneal cultures. Such treatments have included the use of corticosteroids, anti-microbial agents, certain types of saline solutions, etc. Fibronectin preparations have been proposed for the treatment of corneal ulcers, too.

One aspect of the invention resides in the use of a plasmin inhibitor for the manufacture of a pharmaceutical preparation for the treatment of corneal lesions. According to a preferred embodiment of the invention, aprotinin is used as said plasmin inhibitor. The inhibitor may be used in various forms, preferably with a physiologically acceptable carrier. Such carriers may include sterile solutions, ointments and the like. Examples of well known sterile solutions are sterile water, sterile saline solutions, and the like.

A dosage aprotinin composition that can be used in treating a corneal lesion is one containing about 5 IU/ml to about 200 IU/ml of aprotinin. One IU of aprotinin corresponds to about 140 nanograms or about 0.14 $\mu$g aprotinin.

Another aspect of the invention resides in providing a method of determining the presence of corneal lesions comprising taking a sample of a tear fluid from a region suspected to have said lesions and testing said fluid for the presence of a plasmin activity.

The use of the invention in treating corneal lesions is described hereinafter. Tests are provided to show the effect of proteolytic activity. A similar mechanism is believed to apply to various types of lesions of skin and mucous membranes. This form of treatment, inhibition of proteolytic activity, may also be used as a prophylactic to prevent epithelial destruction.

Assay and identification of proteolytic activity in tear fluids. Tear fluid was collected into a glass capillary. Proteolytic activity, using an 8 $\mu$l specimen of tear fluid, was measured by the radial caseinolysis procedure (Saksela, Anal.Biochem.111:276-282), using agarose gel and bovine milk casein as substrate. Human plasmin (20 casein units per mg; Kabi Diagnostica, Stockholm, Sweden) was used as standard. The results are expressed as micrograms of plasmin-like activity per milliliter tear fluid. The advantages of this assay include, the small sample volume needed (minimum 5 $\mu$l), small intra-assay variation (< 5 %) and sensitivity (0.1 $\mu$g plasmin per ml). Repeated freezing and thawing of tear fluid specimens was found to decrease the enzyme activity. Plasminogen activator levels were determined according to Saksela (ibid.) using plasminogen-containing casein-agarose gels and urokinase (50 000 Ploug units/mg; Calbiochem) as standard. Rabbit antibodies to human plasminogen and albumin (DAKO, Copenhagen, Denmark) were used in the identification.

Proteinase inhibitors - Aprotinin (20 000 IU/ml Trasylol®, Bayer), L-cysteine (0.15 M; E. Merck) heparin (2500 IU/ml Medica).

Fibronectin preparation. Fibronectin was purified from human plasma of two healthy volunteers using affinity chromatography on gelatin-agarose (Engvall & Ruoslahti Int J Cancer 20:1-5) and Sephadex® G-25 gel filtration. The final preparation contained 200 $\mu$g/ml fibronectin in 0.15 M arginine-HCl buffer, pH 8.5; human serum albumin, 500 $\mu$g/ml was added as carrier protein. The preparation was devoid of proteolytic activity, was pyrogen-free, free of bacteria and chlamydia and gave negative results in attempted virus isolation. No hepatitis B virus S or HTLV-III antigen were detected. According to sodium dodecyl sulfate polyacrylamide (5-16 %) gel electrophoresis (SDS-PAGE) and immunoblotting with a polyclonal anti-fibronectin rabbit serum, > 95 % of the fibronectin was in intact form.

Zymography. Molecular weights of proteinases were determined using SDS-PAGE under nonreducing condition, extensive washing of the gel with nonionic detergent and overlaying it with casein-agarose. The lytic zones developed within 24-48 h of incubation of +37°.

Patients and control individuals. All patients reported agreed to participate in this preliminary clinical trial and were treated in the Eye Clinic of the Helsinki Central University Hospital. Three healthy females and one male from the laboratory personnel and four cataract patients with no history or signs of ocular inflammatory disease served as controls (see Table 3). The tear fluid was collected from all individuals either by using a Pasteur pipette (spontaneous tearing) or using an 8 $\mu$l capillary tube in cases with low or normal tear secretion.

Proteinase inhibitor and fibronectin treatment. The patients received topical aprotinin (23 or 40 IU/ml), diluted from the stock preparation in sterile saline or commercially obtained wetting agent (Liquifilm Tears; Allergan), 1-2 drops (50 $\mu$l each) at 3 hour intervals. When applied, fibronectin (200 $\mu$g/ml) was administered 2-3 minutes after aprotinin treatment, also using 1-2 drops at a time.

Detailed Description of Using the Invention

Within a period of four months tear fluid specimens of altogether 48 patients with corneal lesions were tested for proteolytic activity. We found that 32 of these were positive. The distribution of the patients in the different diagnosis categories and the results of the tear fluid tests have been summarized in Table 1. A notable finding is the high proportion of patients with therapy-resistant erosion in the group with tear fluid plasmin activity.

Table 1

Tear fluid proteolytic activity in the different patient categories

| Patient group | Plasmin activity | |
| --- | --- | --- |
| | Positive (> 0.1 µg/ml) | Negative (< 0.1 µg/ml) |
| Recurrent or chronic erosion | 7 | 1 |
| Keratitis | 16 | 11 |
| Chronic blepharitis with corneal punctate lesions | 2 | 1 |
| Contact lens lesion | 2 | |
| Contusion | 1 | |
| Mechanical erosion | | 1 |
| Chemical corrosion | 3 | |
| Post-operative cataract or transplantation | 1 | 2 |
| Total number of patients | 32 | 16 |

Antibodies to human plasminogen inhibited the proteolytic activity totally at a 1:25 dilution. The molecular size of the tear fluid proteinase was determined using zymography and were found to comigrate with plasmin ($M_r$ 80 000). No such activity was seen in the zymography of the control tear fluid specimens. In order to clarify whether the plasmin activity was due to elevated levels of plasminogen activator this enzyme was assayed in four patients and was found to be negative (Table 2). In eight control individuals the range of plasminogen activator was 0.6 - 9.8 Ploug units per ml. Aprotinin (an inhibitor of serine proteinases that has been used parenterally in treatment of pancreatis and known to be non-toxic both in vivo and in cell cultures), L-cysteine and heparin (inhibitors of collagenases) were tested as inhibitors. Aprotinin was found to inhibit effectively the proteolytic activity, L-cysteine had a minimal inhibitory effect while heparin had no effect on the activity (Table 3). This formed the basis for the therapeutical approach described below for eighteen patients with proteolytic activity in tear fluid specimens.

Table 2

Plasminogen activator levels in tear fluids specimens of patients and control individuals

| Patient number | Plasminogen activator | |
| --- | --- | --- |
| Treated patients | Date | PU/ml |
| 1 | 22 Oct | < 0.1 |
| 2 | 1 Nov | < 0.1 |
| 3 | 16 Nov | < 0.1 |
| 4 | 25 Nov | < 0.1 |
| Controls | | |
| 19 | 2 Dec | 9.8 |
| 20 | 2 Dec | 0.7 |
| 21 | 8 Dec | 2.4 |
| 22 | 8 Dec | 1.3 |
| 23 | 16 Oct | 0.8 |
| 24 | 18 Oct | 1.2 |
| 25 | 15 Oct | 0.6 |
| 26 | 25 Nov | 1.2 |

The patient numbers refer to Table 4.

Table 3

Effect of inhibitors on the proteolytic activity in tear fluid specimens

| Sample | | Plasmin activity (µg/ml) |
|---|---|---|
| Patient 1 | Tear fluid | 11.6 |
| | + aprotinin (20 IU/ml) | < 0.1 |
| | + L-cysteine (0.075 M) | 10.1 |
| | + heparin (1250 IU/ml) | 11.9 |
| Patient 2 | Tear fluid | 7.0 |
| | + aprotinin (20 IU/ml) | < 0.1 |
| | + L-cysteine (0.075 M) | 7.2 |
| | + heparin 1250 IU/ml | 7.8 |
| Patient 7 | Tear fluid | 7.6 |
| | + aprotinin (10 IU/ml) | < 0.1 |
| Patient 8 | Tear fluid | 6.0 |
| | + aprotinin (10 IU/ml) | < 0.1 |
| | + L-cysteine (0.075 M) | 4.9 |
| | + heparin (1250 IU/ml) | 7.2 |
| Patient 9 | Tear fluid | 6.3 |
| | + aprotinin (10 IU/ml) | < 0.1 |
| | + L-cysteine (0.075 M) | 5.5 |
| | + heparin (1250 IU/ml) | 6.8 |
| Patient 15 | Tear fluid | 4.2 |
| | + aprotinin (10 IU/ml) | < 0.1 |

The patient numbers refer to Table 4.

Table 4. Patients, laboratory data and effect of therapy

| PATIENT/INITIALS/SEX/YEAR OF BIRTH DIAGNOSIS AND MAIN SYMPTOMS | MICROBE | TEAR FLUID PLASMIN ACTIVITY Date | µg/ml | THERAPY AND RESPONSE |
|---|---|---|---|---|
| **Treated patients** | | | | |
| 1  SK     M 1959 Conjunctivitis vernalis, large corneal erosion for 10 weeks. | S. aureus | 22 Oct 30 Oct 10 Dec | 11.6 < 0.1 < 0.1 | Antibiotics and corticosteroids. With fibronectin and aprotinin, recovery started in 2 days. Epithelialization complete in 3 weeks. |
| 2  EA-H   F 1945 Disciform keratitis (history of mechanical trauma), recurrent erosion for 1 week. | None found HSV suspected | 1 Nov 25 Nov | 7.0 < 0.1 | Initially antibiotics and acyclovir with no response. Recovery in 4 days with aprotinin. |
| 3  KA     F 1965 Soft contact lens, giant papillary conjunctivitis, recurrent corneal erosion for 2 days. | None found | 16 Nov 4 Dec | 6.8 < 0.1 | Complete epithelialization in 48 hours following aprotinin therapy. |
| 4  EJ     F 1910 Keratitis sicca, dry eye, leading to corneal ulcer and perforation (20 Sept). | None found | 25 Nov  20 Dec | 5.5  < 0.1 | Little improvement with antibiotics and  soft contact lens. Aprotinin promoted corneal healing which took six weeks. |
| 5  TL     F 1959 Corneal transplantation (keratoconus) and fungal ulcer, symptoms for 2 weeks. | Fungi | 9 Dec | 0.5 | Aprotinin, fibronectin and antifungal drugs for 2 weeks, later fibronectin only since low protease activity. Complete recovery within 4 weeks. |
| 6  SS     M 1932 Corneal ulcer and pseudophacia, irritating structures | None found | 2 Jan | 5.2 | First topical antibiotics and contact lens without improvement. With aprotinin and fibronectin healing in 2 weeks. |

EP 0 223 254 B1

| | | | | | | |
|---|---|---|---|---|---|---|
| 7 | HJ   F 1922<br>Mooren's ulcer | None found | 8 Jan<br>10 Jan<br>16 Jan<br>20 Jan<br>22 Jan<br>27 Jan<br>30 Jan<br>3 Feb | 7.6<br>4.7<br>12.0<br>12.5<br>3.3<br>3.0<br>0.5<br>< 0.1 | With antibiotics and other conventional<br>therapy the illness progressed. With<br>aprotinin (Jan 8) later combined with<br>fibronectin (Jan 22) slow healing<br>starting on Jan 27. |
| 8 | MW   F 1897<br>Deep corneal ulcer. | S. aureus | 6 Jan<br>8 Jan<br>29 Jan<br>31 Jan<br>14 Feb | 6.0<br>4.5<br>0.5<br>0.5<br>< 0.1 | During antibiotic therapy a recidive<br>(Jan 26). After onset of aprotinin (Jan 28),<br>complete healing in 12 days (Feb 9). |
| 9 | AV   F 1911<br>Deep corneal ulcer, ectropium | S. aureus | 16 Jan<br>20 Jan<br>30 Jan<br>13 Feb | 6.3<br>3.0<br>< 0.1<br>< 0.1 | Topical antibiotics, operatio plastica,<br>and aprotinin, rapid epithelialization. |
| 10 | JH   M 1967<br>Giant papillary conjunctivitis for<br>4 days. | None found | 20 Jan<br>22 Jan<br>24 Jan | 10.3<br>2.4<br>< 0.1 | First topical antibiotics. After apro-<br>tinin epithelialization in one day. |
| 11 | MS   F 1905<br>St. post transplantationem corneae,<br>corneal erosion. | None found | 21 Jan<br>30 Jan | 3.1<br>1.9 | Topical antibiotics, partial tarsorrophy<br>and aprotinin, epithelialization in three<br>days following aprotinin therapy. |
| 12 | AP   F 1914<br>Deep corneal abscess | Moraxella | 22 Jan<br>24 Jan<br>6 Feb | 20.0<br>12.4<br>< 0.1 | First antibiotics with little response.<br>Complete epithelialization in 2 weeks<br>after aprotinin. |
| 13 | UM   M 1924<br>Herpetic stromal keratitis leading<br>to cornealerosion and tranplation.<br>Now new erosion. | None found | 31 Jan<br>3 Feb<br>19 Feb | 6.3<br>0.5<br>< 0.1 | Topical acyclovir and antibiotics without<br>response. After aprotinin therapy complete<br>healing in two weeks. |
| 14 | RR   M 1957<br>Chronic blepharitis. | None found | 24 Jan | 3.7 | Topical antibiotics and corticosteroids<br>but chronic course. With aprotinin,<br>condition improved. |

EP 0 223 254 B1

| No. | Patient / Diagnosis | Complications | Date | Value | Treatment |
|---|---|---|---|---|---|
| 15 | RB M 1963 Vernal conjuctivitis, punctate corneal erosion. | None found | 7 Feb | 4.2 | Aprotinin, condition improved in 5 days. |
| 16 | LR M 1924 Corneal burn (cement) | None found | 21 Feb 25 Feb | 3.3 < 0.5 | Antibiotics, aprotinin started on day 2 for 6 days. Complete epithelialization and visual recovery. |
| 17 | MT M 1961 Corneal burn (smoke bomb) | None found | 26 Feb 28 Feb | 13.0 < 0.1 | Antibiotics, aprotinin started on day 2 for 6 days. Topical corticosteroids. Complete epithelialization in 3 days after aprotinin. |
| 18 | MH M 1949 Corneal and conjunctival burn (maleic acid anhydride) | None found | 3 Feb 10 Feb 14 Feb 18 Feb | < 0.1 7.4 (sin) < 0.1 (sin) < 0.1 (sin) | Antibiotics, fibronectin (o.sin., days 1-2 and 7-19; o.dex. days 1-5), aprotinin (o.sin., days 9-30). Epithelialization on day 5 (o.dex.) and on day 20 (o.sin.). |
| Controls | | | | | |
| 19 | F 1921 | Cataract | 2 Dec | < 0.1 | |
| 20 | F 1905 | Cataract | 2 Dec | < 0.1 | |
| 21 | F 1929 | Cataract | 8 Dec | < 0.1 | |
| 22 | F 1932 | Cataract | 8 Dec | < 0.1 | |
| 23 | F 1950 | (Healthy nurse) | 16 Oct | < 0.1 | |
| 24 | F 1946 | (Scientist) | 18 Oct | < 0.1 | |
| 25 | M 1958 | (Scientist) | 15 Oct | < 0.1 | |
| 26 | F 1958 | (Technician) | 25 Nov | < 0.1 | |

The first patient (number 1 in Tables 3 and 4) with a chronic corneal erosion resistant to conventional therapy for 10 weeks (antibiotics, corticosteroids) was initially treated with topical fibronectin starting on October 16. One day later the corneal erosion had an altered appearance. There was a thin layer of abnormal and cloudy epithelium at the bottom of the crater. A small epithelial scraping on October 19 confirmed the presence of epithelial cells in the wound. Tear fluid analysis revealed high plasmin activity. Topical fibronectin was, therefore, on October 22 combined with topical proteinase inhibitor (aprotinin).

8

There was an immediate dramatic improvement in this condition so that on October 30 he had already visual acuity 0.5. On January 15 his visual acuity was 0.7 and the epithelium intact.

After the success with this index case and similar experience with the first few additional patients we adopted the following therapeutical regimen. Patients with corneal ulcers were first treated for four days with conventional therapy including appropriate antimicrobial drugs according to the laboratory findings. If no response was seen and if plasmin was detected in the tear fluid, aprotinin therapy was initiated. Following this regimen, six of the patients had been previously treated with conventional therapy for 3-10 weeks without response (patients 1, 4, 5, 6, 7 and 18). In some cases with low plasmin activity in the initial or later tear fluid specimens, aprotinin was combined with topical fibronectin. In addition, in certain patients such as in patient 18 with bilateral acid burn of the cornea no plasmin was detected immediately after injury. The right eye of the patient was initially treated with topical fibronectin with clear beneficial effect and epithelial healing. However, when fibronectin was 7 days later applied to the left eye, no such therapeutic effect was observed. The tear fluid was reanalyzed and now showed plasmin. Aprotinin therapy was initiated and led to rapid epithelialization. However, a large proportion of patients with therapy-resistant corneal lesions of various categories (Table 2) showed plasmin activity and the above regimen was followed. In all eighteen patients treated so far (Table 4) this therapy has led to complete corneal healing. The longest treatment with aprotinin eye drops (patient 4 with dry eyes and spontaneous perforation) lasted five weeks and yielded good results without corneal or other complications.

Occular patching is the current treatment of choice for corneal erosions. During this study it was thought that in a few patients patching for more than one day was occasionally followed by an increase of the tear fluid proteolytic activity. This was observed for patients 1, 7, 18 and another patient not listed in Table 4.

All three patients with chemical corrosion (patients 16-18) had plasmin in tear fluid. In the most severe case (patient 18) the activity appeared several days after the injury, correlating with cessation of epithelialization. With aprotinin therapy the activity disappeared in epithelialization resumed.

The plasmin activity in tear fluid was inhibited by both antibodies to human plasminogen and aprotinin. This finding and the comigration of the proteolytic activity with human plasmin, indicates that plasmin is the principal tear fluid proteinase. The presence of collagenase activity in corneal ulcerations has been previously recognized. The main drugs to inhibit collagenolytic activity, thought to destruct corneal tissue, have been L-cysteine, EDTA and heparin. This type of treatment was used at first in patients 1, 3 and 4 but with little or no clinical effect. The cornea of patient 4 perforated spontaneously during topical L-cysteine therapy in the absence of detectable microbial pathogens, probably due to proteolytic activity. These inhibitors of collagenase had also very little effect on the proteolytic activity of tear fluid of patients 1, 2, 8 and 9 in vitro. In keratitis caused by the opportunistic pathogen Serratia marcescens the $M_r$ 56 000 bacterial metalloproteinase is thought to be a major pathogenic factor. On the basis of our results it seems possible that therapeutic intervention with proteinase inhibitor could be beneficial also in these patients.

Similar proteolytic activation and destruction as described here for corneal lesions, and predicted previously, conceivably operate in various lesions of skin and mucous membranes, such as those caused by trauma, infections and chronic disease processes.

## Claims

1. Use of a plasmin inhibitor for the manufacture of a pharmaceutical preparation for the treatment of corneal lesions.

2. The use as claimed in claim 1, wherein said plasmin inhibitor is aprotinin.

3. The use as claimed in claim 2, wherein said preparation comprises about 5 IU/ml to about 200 IU/ml aprotinin.

4. A method of determining the presence of corneal lesions comprising taking a sample of a tear fluid from a region suspected to have said lesions, and testing said fluid for the presence of a plasmin activity.

## Revendications

1. Utilisation d'un inhibiteur de la plasmine pour la fabrication d'une préparation pharmaceutique destinée au traitement de lésions de la cornée.

2. Utilisation telle que revendiquée à la revendication 1 selon laquelle l'inhibiteur de la plasmine est l'aprotinine .

3. Utilisation telle que revendiquée à la revendication 2 selon laquelle la préparation comprend de 5 UI/ml environ à 200 UI/ml environ d'aprotinine.

4. Procédé pour la détermination de la présence de lésions de la cornée comprenant le prélèvement d'un échantillon de fluide lacrymal d'une région suspectée d'avoir ces lésions et l'épreuve de ce fluide pour détecter la présence d'une activité de la plasmine.

**Ansprüche**

1. Verwendung eines Plasmininhibitors zur Herstellung einer pharmazeutischen Zubereitung für die Behandlung von Verletzungen der Hornhaut des Auges.

2. Verwendung nach Anspruch 1, wobei der Plasmininhibitor Aprotinin ist.

3. Verwendung nach Anspruch 2, wobei die Zubereitung etwa 5 IU/ml bis etwa 200 IU/ml Aprotinin enthält.

4. Verfahren zur Feststellung von Verletzungen der Hornhaut des Auges, bei dem eine Probe der Tränenflüssigkeit von einem Bereich genommen wird, in dem Verletzungen vermutet werden, und bei dem die Flüssigkeit auf die Anwesenheit einer Plasminaktivität untersucht wird.